# EUROPEAN PATENT APPLICATION

(11) **EP 2 067 865 A1**
(43) Date of publication of application: **10.06.2009**
(21) Application number: 08164992.3
(22) Date of filing: 24.09.2008
(51) Int. Cl.: C12Q 1/00, G01N 33/487

(54) **Biochemical test system, measurement device, and biochemical test strip**

(30) Priority: 07.12.2007 TW 96146711; 01.02.2008 TW 97202289 U; 12.05.2008 TW 97208206 U
(71) Applicant: Apex Biotechnology Corporation, Hsinchu City 300 (TW)
(72) Inventor: Yang, Mon Wen, 300, Hsinchu City (CN); Chu, Ching-Yuan, 300, Hsinchu City (CN); Lin, Yueh-Hui, 300, Hsinchu City (CN); Hsu, Ming-Chang, 300, Hsinchu City (CN); Wang, Jui-Ping, 300, Hsinchu City (CN); Shen, Thomas Y. S., 300, Hsinchu City (CN)
(74) Representative: Ketelaars, Maarten F.J.M.

(57) **Abstract**

A biochemical test system, a measurement device, and a biochemical test strip are provided. The biochemical test strip includes an insulating substrate, an electrode system disposed on the insulating substrate, and an identifying unit disposed on one side of the insulating substrate. The identifying unit includes N components, and at least one of the N components includes a groove. It should be noted that the term "N" in this specification is an integer greater than two. The measurement device includes a microprocessor and a connector, wherein the connector is coupled to the identifying unit for receiving a signal of an identification code corresponding to the identifying unit, and the microprocessor is coupled to the connector for receiving the signal from the connector.

## Description

### Cross Reference to Related Applications

This application claims the benefit of the following three applications: Taiwan Patent Application No. 096146711 filed on December 7, 2007, Taiwan Patent Application No. 097202289 filed on February 1, 2008, and Taiwan Patent Application No. 097208206 filed on May 12, 2008. All the above-cited references are fully incorporated herein by reference.

### Field of Invention

The present invention relates to a biochemical test system, a measurement device, and a biochemical test strip, and more particularly, to a biochemical test system, a measurement device, and a biochemical test strip, with an auto correction to dismiss the need for code card.

### Background of the Invention

With the advance of the medical science and the rising concept from the modem people about health care, the Point-of-Care (POCT) has been widely available to the market. Such kind of self-testing products, such as blood glucose monitor, electrical ear thermometer, and electrical sphygmomanometer, tend to be fast, cheap, small and getting rid of professional help for the operation. In such field, the use of the biochemical test strip is a well-versed skill, especially for the popular application of monitoring the blood glucose.

In the conventional biochemical test system, every batch of biochemical test strips has been defined a unique parameter during the production process. Therefore, before using a batch of biochemical test strip for a test on a measurement device, a code card is needed to calibrate the measurement device, as disclosed in US Patent No. 5,582,697 and PCT Publication No. WO00/33072. However, to manufacture the code card will increase the production cost and the labor power, also the correction error and the data measurement error occur frequently because users forget to insert the code card, or use a wrong code card, or the code card is lost.

To solve the inconvenience with using the code card, US Patent No. 6,814,844 disclosed an identification method by using bar codes. FIG. 1 indicates a conventional test strip 100 comprising a conductive electrode set 110 with a plurality of electrodes insulated from each other, and a bar code 120 disposed between the conductive electrode set 110. The bar code 120 is a bar code pattern formed on the substrate by laser ablation, specifically, the bar code pattern is formed by using a high-energy pulsed laser to bombard the surface of a gold target material coated on the substrate, so that a portion of the gold target material is removed, and the desired bar code pattern is formed. However, as disclosed in US patent 6,814,844, the identification methods for bar code 120 are optical measurement systems, using CCD or LED for detection, for example. Moreover, the reproduction and the accuracy highly depend on the surface condition of the target material, therefore there is not only a limitation to the fabrication, but also an increase in the production cost.

Accordingly, it is advantageous to have a biochemical test system capable of avoiding the code card correction and keeping the production yield and the test accuracy.

### Summary of the Invention

In view of the problems existing in the prior art, the present invention provides an auto-correction biochemical test system capable of eliminating the use of a discrete code card, and reducing the production failure rate.

According to an aspect of the present invention, a biochemical test system including a biochemical test strip and a measurement device is provided. The biochemical test strip includes an insulating substrate, an electrode system disposed on the insulating substrate, and an identifying unit disposed on one side of the insulating substrate. The identifying unit includes N components, and at least one of the N components includes a groove. It should be noted that the term "N" in this specification is an integer greater than two. The measurement device includes a microprocessor and a connector, wherein the connector is coupled to the identifying unit for receiving a signal of an identification code corresponding to the identifying unit, and the microprocessor is coupled to the connector for receiving the signal from the connector.

According to another aspect of the present invention, a measurement device is provided. The measurement device is used with a biochemical test strip, wherein the biochemical test strip includes an insulating substrate, an electrode system disposed on the insulating substrate, and an identifying unit disposed on one side of the insulating substrate. The identifying unit includes N components, and at least one of the N components includes a groove. The measurement device includes a connector and a microprocessor. The connector electrically couples to the identifying unit for receiving a signal of an identification code corresponding to the identifying unit. The microprocessor couples to the connector for receiving the signal from the connector. According to another aspect of the present invention, a biochemical test strip including an insulating substrate, an electrode system disposed on the insulating substrate, and an identifying unit disposed on a predetermined region of the insulating substrate. The identifying unit includes N components, and at least one of the N components includes a groove. An identification code for the biochemical test strip is decided according to the location of the groove.

The other aspects of the present invention, part of them will be described in the following description, part of them will be apparent from description, or can be known from the execution of the present invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### Brief Description of the Pictures

The foregoing aspects and many of the attendant advantages of this invention will become more readily appreciated as the same becomes better understood by reference to the following detailed description, when taken in conjunction with the accompanying pictures, wherein:

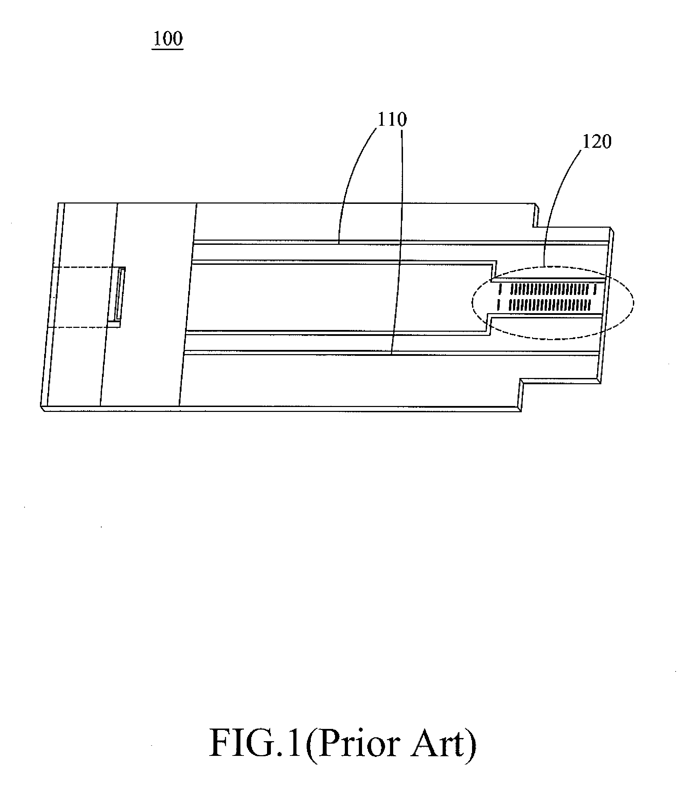

FIG. 1 illustrates a conventional biochemical test strip;
FIG. 2 illustrates a biochemical test strip according to an embodiment of the present invention;
FIG. 3 illustrates an explosive view of the biochemical test strip shown in FIG. 2;
FIG. 4 illustrates a biochemical test strip according to another embodiment of the present invention;
FIG. 5 illustrates an explosive view of the biochemical test strip shown in FIG. 4;
FIG. 6 illustrates a biochemical test strip according to still another embodiment of the present invention;
FIG. 7 illustrates an explosive view of the biochemical test strip shown in FIG. 6;
FIGS. 8 and 9 are the biochemical test strips according to different embodiments of the present invention;
FIG. 10 is biochemical test strip according to another embodiment of the present invention;
FIGS. 11 and 12 are biochemical test strips according to different embodiments of the present invention;
FIGS. 13 and 14 are biochemical test strips according to different embodiments of the present invention;
FIG. 15 is a block diagram of a biochemical test system according to an embodiment of the present invention;
FIGS. 16A and 16B illustrate a detecting unit and a sensing unit of a connector respectively;
FIG. 17 is a block diagram of a biochemical test system according to an embodiment of the present invention;
FIGS. 18A, 18B, and 18C are illustrative diagrams of the connectors of the biochemical test strips shown in FIGS. 11,13 and 14 respectively; and
FIG. 19 is a block diagram of a biochemical test system according to an embodiment of the present invention.

### Detailed Description of the Invention

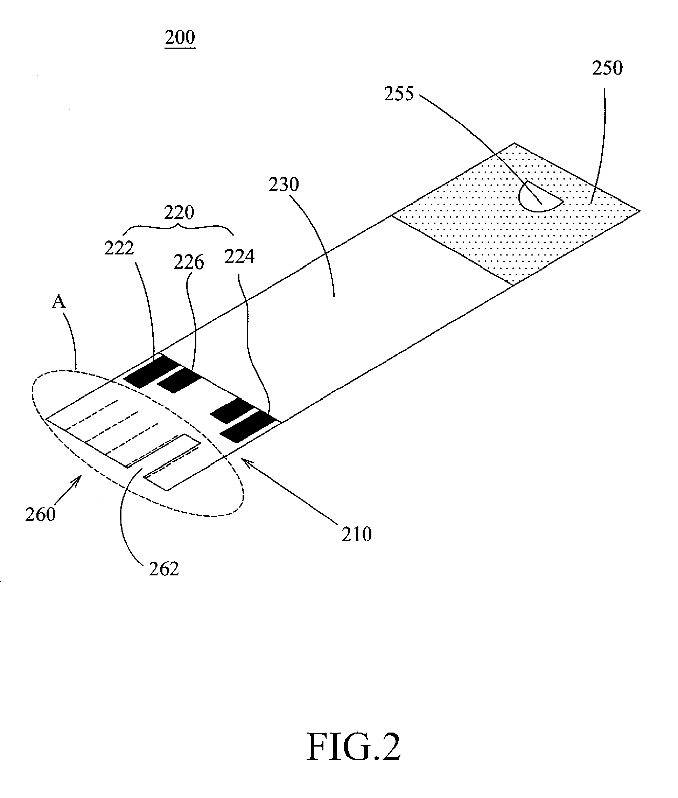

The present invention discloses a biochemical test system, a measurement device, and a biochemical test strip, which eliminate the need of a discrete code card, provide easy operation, prevent the users from forgetting to insert the code card or use a wrong code card, and reducing the possibility of errors during the production process. The present invention will be described more fully hereinafter with reference to the FIGS. 2-19. However, the devices, elements, and methods in the following description are configured to illustrate the present invention, and should not be construed in a limiting sense.

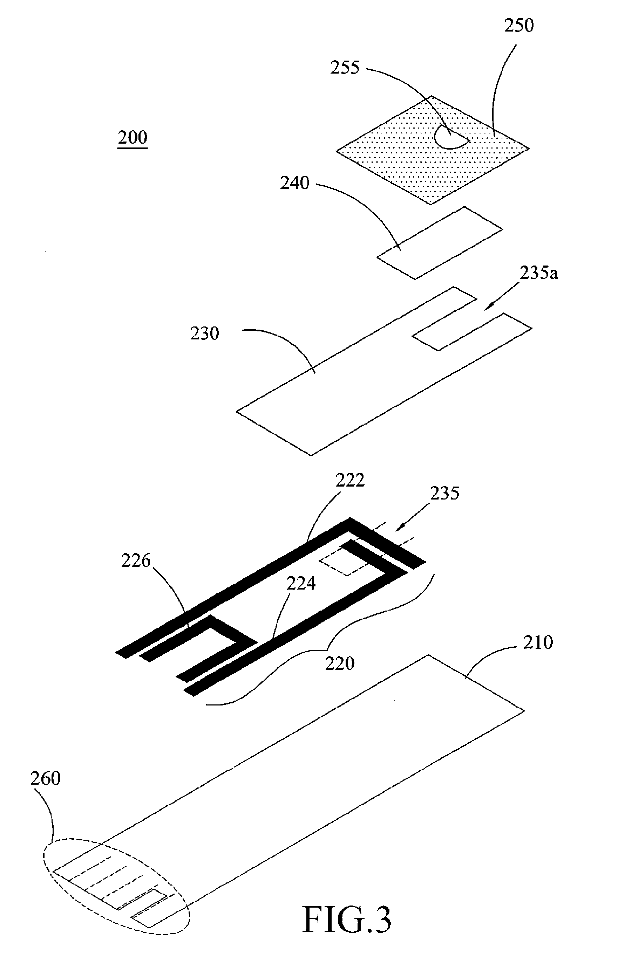

FIG. 2 illustrates a biochemical test strip 200 according to an embodiment of the present invention, and FIG. 3 illustrates an explosive view of the biochemical test strip 200 shown in FIG. 2. The biochemical test strip 200 of the present invention includes an insulating substrate 210, a conductive layer 220, an insulating layer 230, a cover 250, and an identifying unit 260 formed by the insulating substrate 210.

The insulating substrate 210 is electrically insulating, and its material can include but not limit to: polyvinylchloride (PVC), glass fiber (FR-4), polyester, bakelite, polyethylene terephthalate (PET), Polycarbonate (PC), polypropylene (PP), polyethylene (PE), polystyrene (PS), or ceramic material.

As shown in FIGS. 2 and 3, the identifying unit 260 is formed by a predetermined region A of the insulating substrate 210. For example, the predetermined region A is disposed on one side of the insulating substrate 210, and the identifying unit 260 is formed depending on whether there is at least one groove 262 in the predetermined region A of the insulating substrate 210. An identification code corresponding to the identifying unit 260 is decided according to the location of the groove 262. For example, the components of the identifying unit 260 can be formed by dividing the predetermined region A of the insulating substrate 210 into a plurality of subregions based on the desired number of identification code, without coating any metal pattern thereon. When the identifying unit 260 includes groove(s), the identification code represented by the identifying unit 260 can be defined according to the number and the location of the groove(s). In other words, each subregion can be a desired location of the groove 262, and the size of the groove 262 may vary with the size of each subregion, preferably, equals to the size of one subregion. As shown in FIGS. 2 and 3, in this embodiment, the predetermined region A of the insulating substrate 210 is divided into six subregions to form the identifying unit 260, and a groove 262 is formed in one of the subregions. The groove 262 of the present invention can be a channel, an indentation, or a hole penetrating the insulating substrate, and also can be a channel, an indentation, or a hole without penetrating the insulating substrate. In this embodiment, the groove 262 is a rectangular indentation penetrating the insulating substrate 210, but it should be noted that the present invention is not limited to the shape of the components of the identifying unit 260. The operation of the identifying unit 260 will be described below.

The conductive layer 220 includes at least one electrode system, and the electrode system includes at least one working electrode and one reference electrode. In one embodiment, the conductive layer 220 includes a working electrode 222, a reference electrode 224, and a sensing electrode 226, which are insulated from one another.

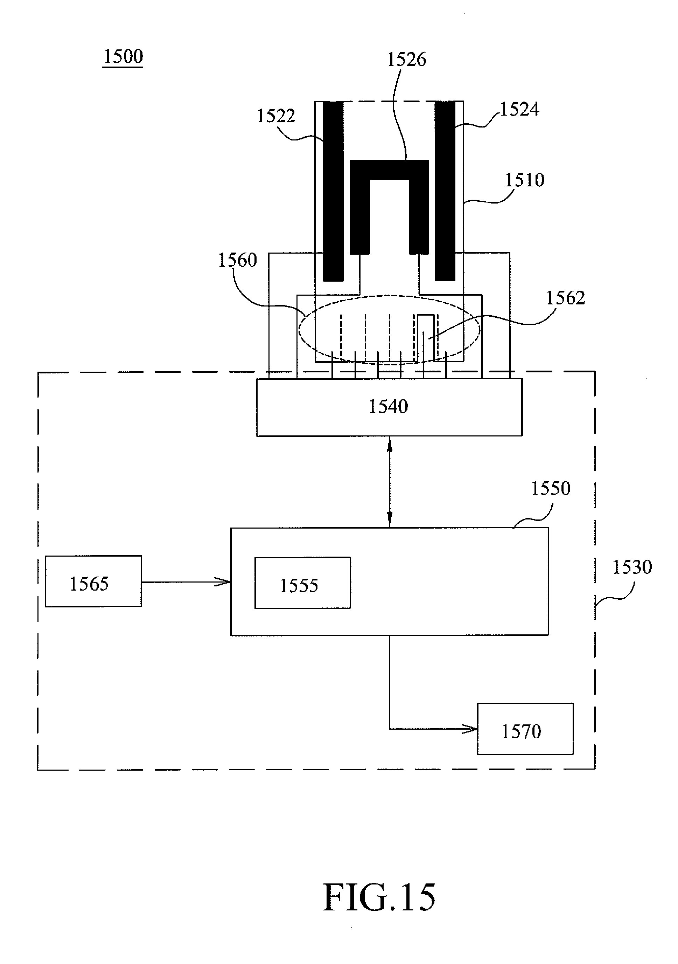

The conductive layer 220 can be any known conductive material such as carbon paste, gold-silver paste, copper paste, carbon/silver paste, or other similar material and the combination thereof. In an embodiment, the conductive layer 220 includes a conductive silver paste layer and a conductive carbon paste layer disposed on the conductive silver paste layer. In this embodiment, the sensing electrode 226 is disposed between the working electrode 222 and the reference electrode 224 and configured to detect an electrical connection between the biochemical test strip 200 and a measurement device (as 1530 shown in FIG. 15). When the biochemical test strip 200 is inserted into the measurement device, a loop is formed between the sensing electrode 226 and the measurement device to activate the measurement device. In fact, it's sufficient as each electrode in a reaction area follows the arrangement order as mentioned above, and the electrodes are electrically insulated from one another. The present invention is not limited to the arrangement method for the working electrode 222, the reference electrode 224 and the sensing electrode 226 illustrated in the embodiment, or the number of electrodes used. Additional electrodes can be added according to different application need.

The insulating layer 230 is disposed on the conductive layer 220, and includes an indentation 235a to expose a part of the conductive layer 220. It's sufficient for the indentation 235a to expose part of the working electrode 222 and part of the reference electrode 224. The present invention is not limited to the shape of the indentation 235a. Besides, the insulating layer 230 also exposes another part (not shown) of the conductive layer 220 so that the conductive layer 220 can electrically connect to the measurement device (as 1530 shown in FIG. 15). The material of the insulating layer 230 can include but is not limited to: PVC insulating tape, PET insulating tape, thermal drying insulating paint or ultraviolet drying insulating paint.

The cover 250 is disposed on the insulating layer 230, covering the indentation 235a. The indentation 235a forms a sample space (i.e. reaction area 235) with capillary attraction between the insulating substrate 210 and the cover 250. When the area of the sample space is fixed, its volume depends on the thickness of the insulating layer 230. Generally, the thickness of the insulating layer 230 is between 0.005 and 0.3 millimeter, but not limited thereto. Furthermore, an insulating layer 230 with a precut indentation 235a can be disposed on the insulating substrate 210 and the conductive layer 220. Alternatively, the insulating layer 230 can be formed directly on part of the insulating substrate 210 and the conductive layer 220 by a printing method, which is defined with the indentation 235a and exposes the contact area to be coupled with the measurement device.

The biochemical test strip 200 of the present invention further includes a reaction layer 240 with the ability to identify specified organism material or signal. The material of the reaction layer 240 can be varied with sample types, such as an oxidoreductase for reacting with the sample. Generally, the reaction layer 240 should at least cover part of the working electrode 222.

The cover 250 of the present invention can be transparent or translucent material, so that the users may check whether the sample has been disposed on the reaction area to avoid a false result. The lower surface of the cover 250 close to the reaction area can be coated with a hydrophile material to enhance the capillary action on the inner surface of the reaction area. In this way the sample can be conducted to the reaction area more quickly and efficiently. The cover 250 further includes a vent 255 corresponding to the reaction area for expelling the air inside the reaction area to enhance the capillary action. Generally, the vent 255 is near the end side of the reaction area. The present invention is not limited to the shape of the vent 255. For example, the vent 255 can be a circle, an ellipse, a rectangle, and a rhombus etc.

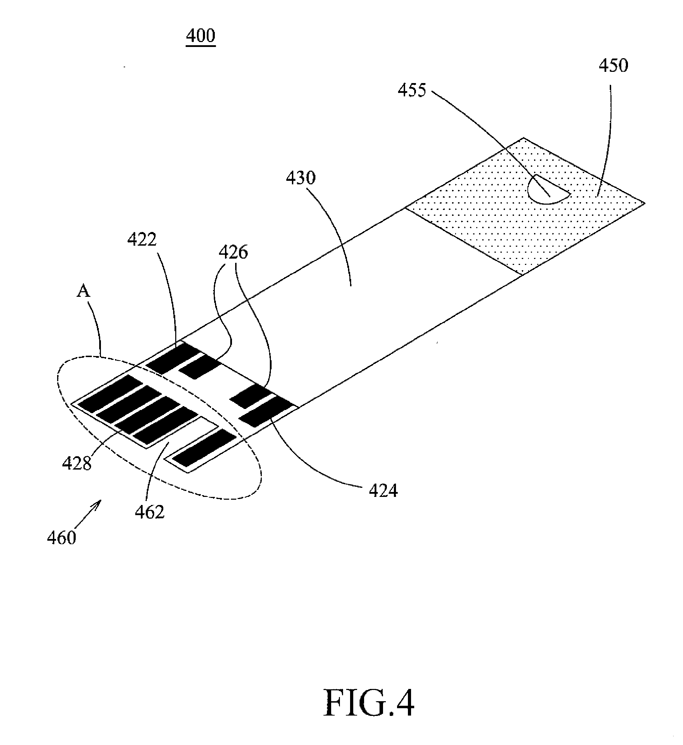

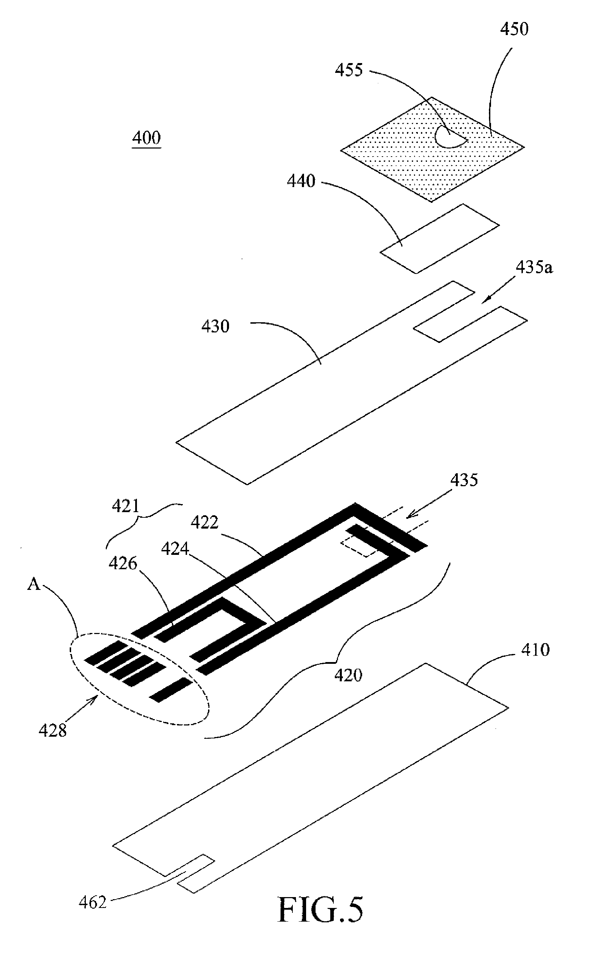

FIG. 4 illustrates a biochemical test strip 400 according to another embodiment of the present invention, and FIG. 5 illustrates an explosive view of the biochemical test strip 400 shown in FIG. 4. The biochemical test strip 400 of the present invention includes an insulating substrate 410, a conductive layer 420, an insulating layer 430 with an indentation 435a, a reaction layer 440, and a cover 450 with a vent 455, wherein the conductive layer 420 includes an electrode system 421 and a pattern code 428. It should be understood that the pattern code 428 is also consisted of conductive material. In an embodiment, the identifying unit 460 is formed by a predetermined region A of the insulating substrate 410 and the pattern code 428 thereon, and the predetermined region A includes at least a groove 462 which can either penetrate or not penetrate the insulating substrate 410. The electrode system 421 includes a working electrode 422, a reference electrode 424, and a sensing electrode 426, which are insulated from one another. Comparing with the biochemical test strip 200 in FIGS. 2 and 3, the identifying unit 460 of the biochemical test strip 400 in the FIGS. 4 and 5 further includes the pattern code 428 formed by the conductive material, whereby the identification code represented by the identifying unit 460 can be retrieved by electrically connecting the pattern code 428 directly to a measurement device (as 1530 shown in FIG. 15). The functions of other elements of the biochemical test strip 400 are same as that in FIGS. 2 and 3, so the detail description thereof is omitted.

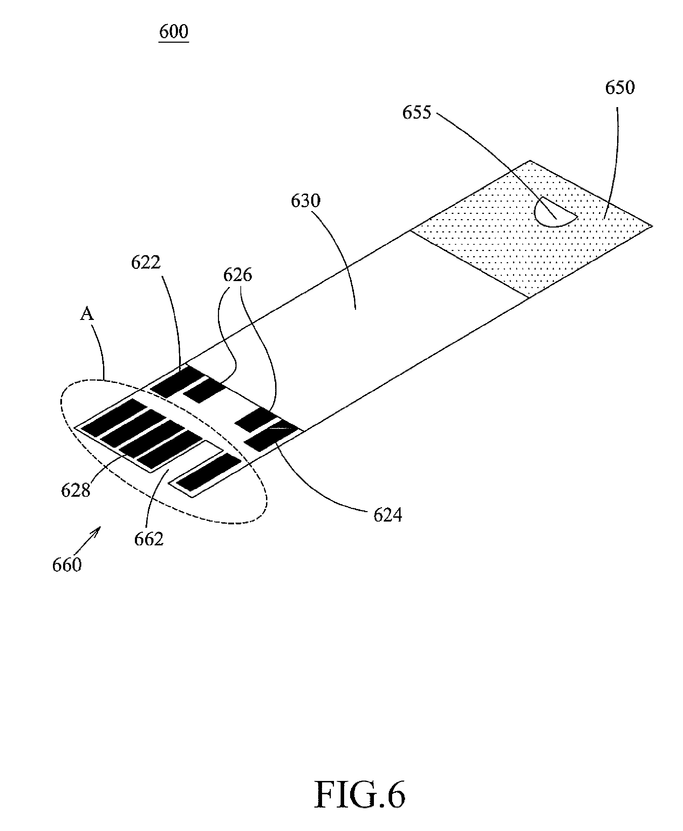

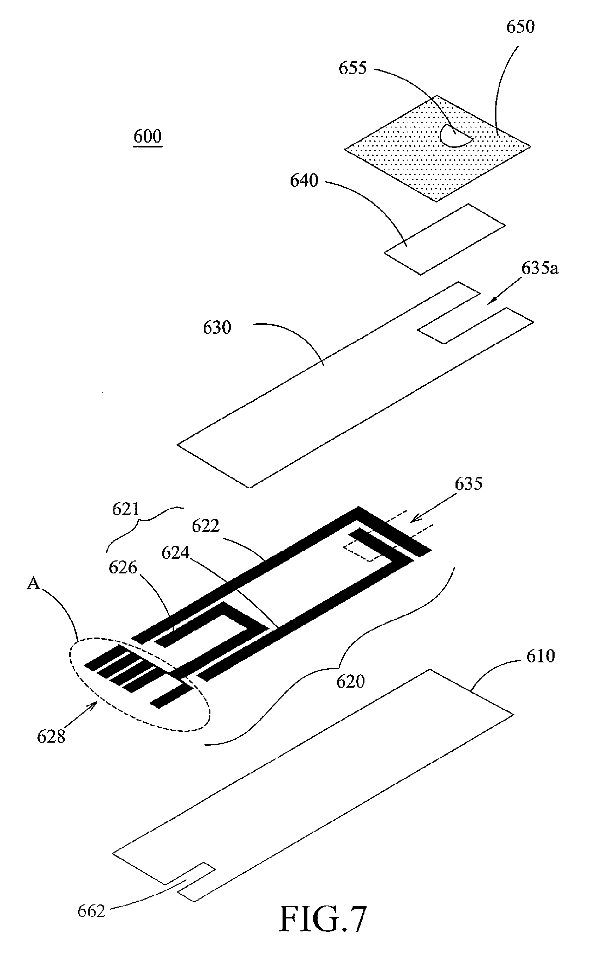

FIG. 6 illustrates a biochemical test strip 600 according to still another embodiment of the present invention, and FIG. 7 illustrates an explosive view of the biochemical test strip 600 shown in FIG. 6. The biochemical test strip 600 of the present invention includes an insulating substrate 610, a conductive layer 620, an insulating layer 630 with an indentation 635a, a reaction layer 640, and a cover 650 with a vent 655, wherein the conductive layer 620 includes an electrode system 621, a pattern code 628, and a linking unit 629. A predetermined region A of the insulating substrate 610 includes at least a groove 662 which can either penetrate or not penetrate the insulating substrate 610. The identifying unit 660 is formed by the predetermined region A of the insulating substrate 610 and the pattern code 628 thereon. In this embodiment, the electrode system 621 includes a working electrode 622, a reference electrode 624, and a sensing electrode 626 insulated from one another, wherein one side of the linking unit 629 connects with the pattern code 628, and the other side of the linking unit 629 connects with the sensing electrode 626, for providing a common ground for both the pattern code 628 and the sensing electrode 626. The functions of other elements of the biochemical test strip 600 are same as that in FIGS. 2 and 3, so the detail description thereof is omitted.

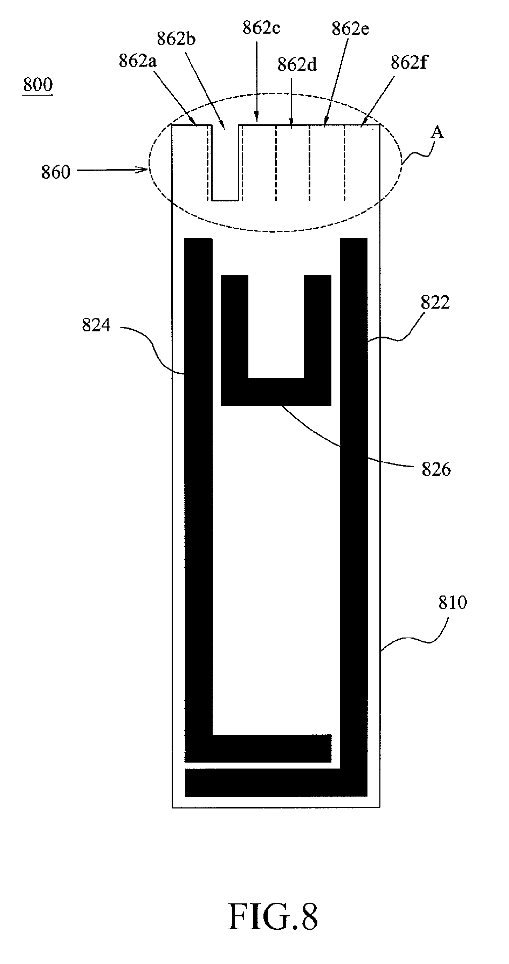

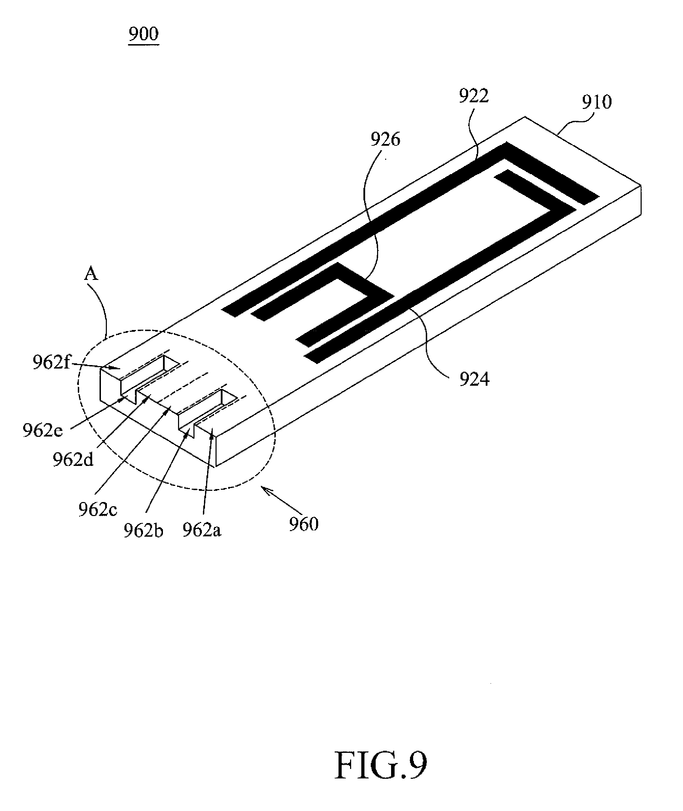

FIGS. 8 and 9 illustrate two biochemical test strips 800 and 900 according to different embodiments of the present invention, and it should be noted that the insulating layer and the cover are not shown for simplification. The biochemical test strip 800 includes an insulating substrate 810, a working electrode 822, a reference electrode 824, and a sensing electrode 826, which are insulated from one another. A part of the insulating substrate 810 (i.e. the predetermined region A) constructs an identifying unit 860. The identifying unit 860 includes a plurality of components which are formed by dividing the predetermined region A into a plurality of subregions. In this embodiment, the identifying unit 860 includes six components (i.e. 862a, 862b, 862c, 862d, 862e, and 862f), wherein one component 862b is formed as a groove, and other components are formed by the remaining areas of the predetermined region A. The component 862b is preferably, but not limited to, an indentation penetrating the insulating substrate 810. In another embodiment, referring to FIG. 9, the configuration of the biochemical test strip 900 is similar to that in FIG. 8, which includes an insulating substrate 910, a working electrode 922, a reference electrode 924, and a sensing electrode 926. The identifying unit 960 includes two components 962b and 962e formed as grooves, and four components 962a, 962c, 962d, 962f formed by the remaining areas of the predetermined region A. In this embodiment, the components 962b and 962e are channels not penetrating the insulating substrate 910. In other words, when performing a cutting or a punching process on the insulating substrate 910 to produce a plurality of biochemical test strips with defined identifying unit 960, only part of the insulating substrate 910 is removed such that part of the predetermined region (i.e. the components 962b and 962e) is thinner than the original substrate 910. Therefore, when the biochemical test strip 800 or 900 is inserted into a measurement device, the corresponding connection between the identifying units 860, 960 and the measurement device, which is based on the number and the location of the groove(s), can be detected, and therefore the biochemical test strip 800 or 900 can be identified by the measurement device. It should be noted that although the grooves 962b and 962e in FIG. 9 are shown to be formed on the top surface of the insulating substrate 910 (i.e. on the same side as the electrodes 922, 924, and 926), they can also be formed on the bottom surface of the insulating substrate 910, as long as the measurement device can identify the identification code represented by the identifying unit 960.

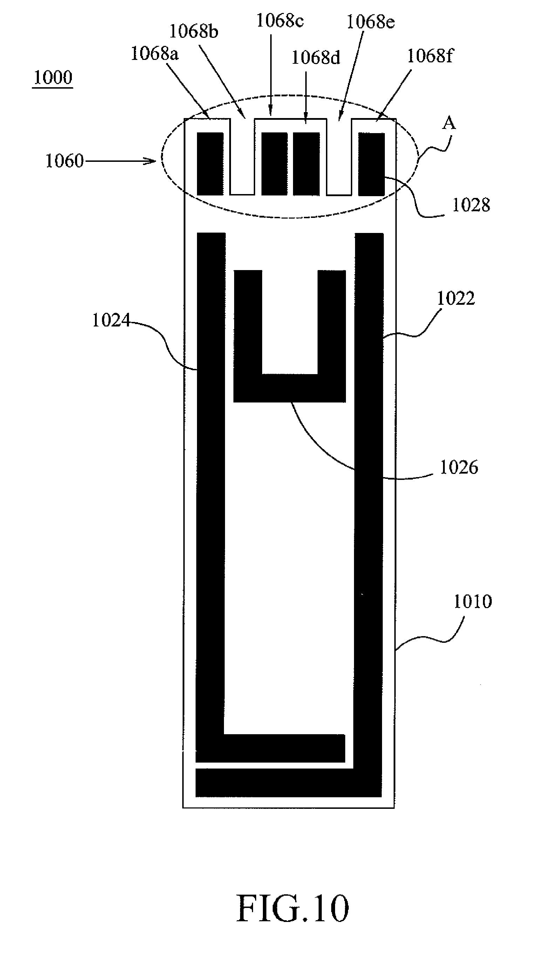

FIG. 10 is biochemical test strip 1000 according to another embodiment of the present invention. The biochemical test strip 1000 includes an insulating substrate 1010, a working electrode 1022, a reference electrode 1024, a sensing electrode 1026, and an identifying unit 1060. The identifying unit 1060 may include a plurality of components, which are comprised of a pattern code 1028 and a plurality of subregions in a predetermined region A of the insulating substrate 1010. In this embodiment, the identifying unit 1060 includes a plurality of components 1068a, 1068b, 1068c, 1068d, 1068e, and 1068f, and the components 1068b and 1068e are grooves penetrating the insulating substrate. Therefore, when the biochemical test strip 1000 is inserted into the measurement device, the grooves (such as components 1068b, or 1068e) of the identifying unit 1060 can't form an electrical connection with the measurement device, and therefore the biochemical test strip 1000 can be identified by the measurement device. It should be noted that although the pattern code 1028 in FIG. 10 are shown to be formed on the same side as the electrodes 1022, 1024, and 1026, they can also be formed on the opposite side of the insulating substrate 1010 and operate in coordination with a measurement device having a corresponding structure capable of detecting the electrical connection between the identifying unit 1060 and the measurement device.

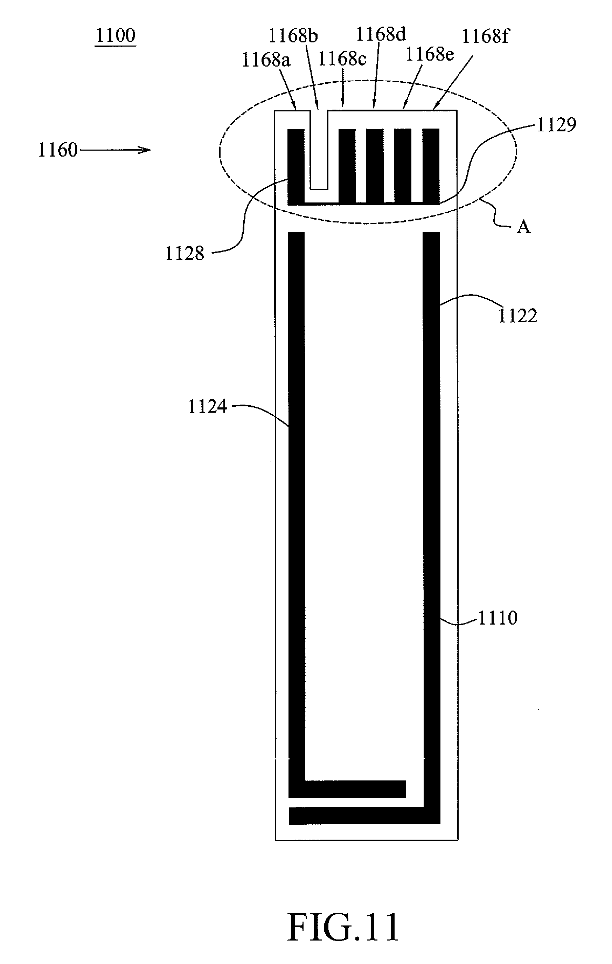

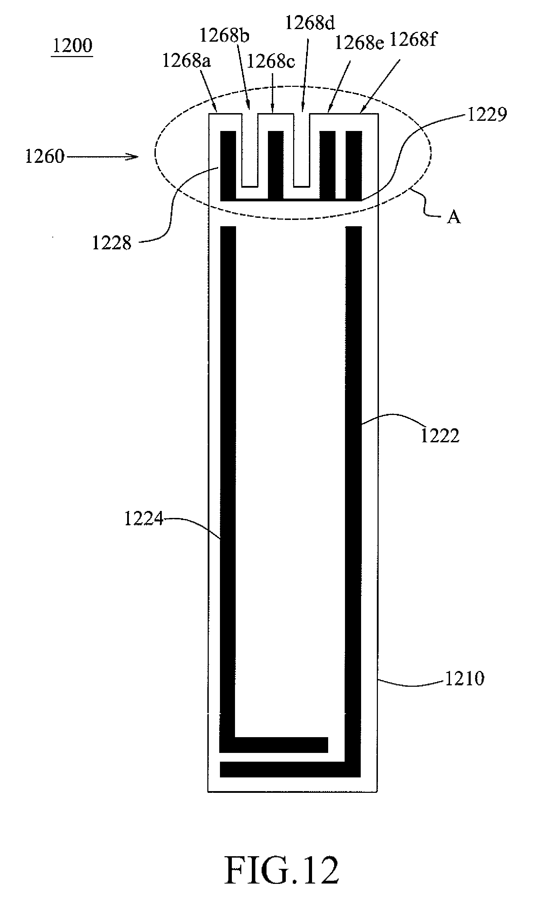

FIGS. 11 and 12 are two biochemical test strips 1100 and 1200 according to different embodiments of the present invention. The biochemical test strip 1100 includes an insulating substrate 1110, a working electrode 1122 and a reference electrode 1124 insulated from each other, an identifying unit 1160, and a linking unit 1129. The identifying unit 1160 includes six components 1168a, 1168b, 1168c, 1168d, 1168e, and 1168f, which are formed by a predetermined region A of the insulating substrate 1110 and a pattern code 1128 thereon. One side of the linking unit 1129 connects to one end of each of the six components of the identifying unit 1160 to provide a common ground for these six components. In this embodiment, the component 1168b is a groove penetrating the insulating substrate 1110. Referring to FIG. 12, the biochemical test strip 1200 includes an insulating substrate 1210, a working electrode 1222 and a reference electrode 1224 insulated from each other, an identifying unit 1260, and a linking unit 1229. The identifying unit 1260 includes six components 1268a, 1268b, 1268c, 1268d, 1268e, and 1268f, which are formed by a predetermined region A of the insulating substrate 1210 and a pattern code 1228 thereon. One side of the linking unit 1229 connects to one end of each of the six components of the identifying unit 1260 to provide a common ground for these six components. In the embodiment shown in FIG. 12, the components 1268b and 1228d are grooves penetrating the insulating substrate 1210. When the biochemical test strip 1100 or 1200 is inserted into a measurement device, the grooves (such as components 1168b, 1268b or 1268d) of the identifying unit 1160 or 1260 can't form an electrical connection with the measurement device, therefore the biochemical test strip 1100 or 1200 can be identified by the measurement device and measured by selecting corresponding correction parameters or modes.

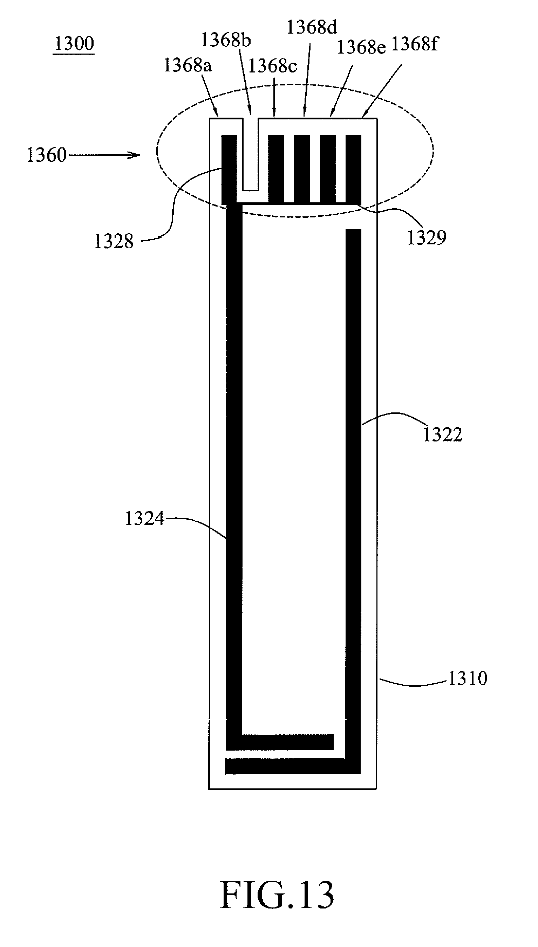

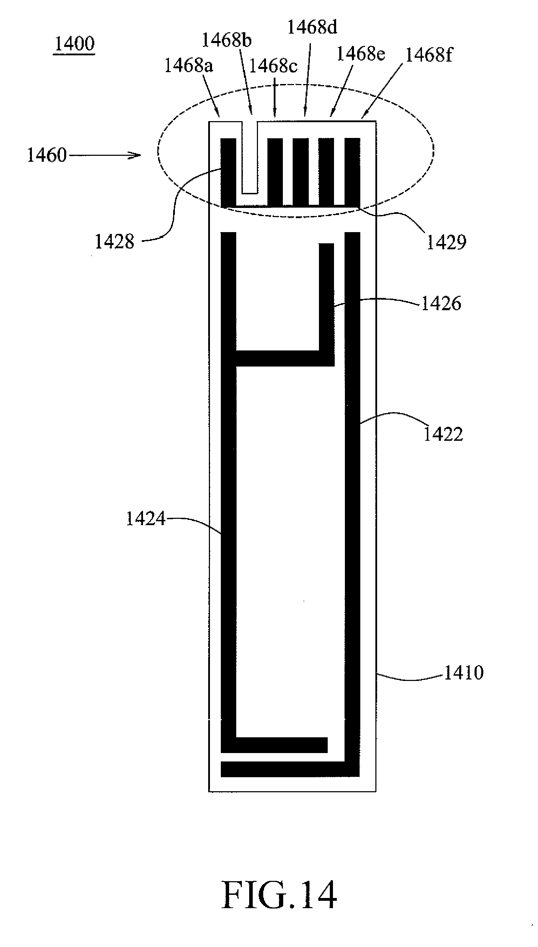

FIGS. 13 and 14 are two biochemical test strips 1300 and 1400 according to different embodiments of the present invention, which illustrate other connection configurations of the linking unit and the electrodes. Referring to FIG. 13, the biochemical test strip 1300 includes an insulating substrate 1310, a working electrode 1322 and a reference electrode 1324 insulated from each other, an identifying unit 1360, and a linking unit 1329. The identifying unit 1360 includes six components 1368a, 1368b, 1368c, 1368d, 1368e, and 1368f. In this embodiment, one side of the linking unit 1329 connects to one end of each of the components 1368a, 1368b, 1368c, 1368d, 1368e, and 1368f, and the other side of the linking unit 1329 connects to the reference electrode 1324, for providing a common ground for the six components 1368a, 1368b, 1368c, 1368d, 1368e, and 1368f and the reference electrode. Referring to FIG. 14, the biochemical test strip 1400 includes an insulating substrate 1410, a working electrode 1422, a reference electrode 1424, a sensing electrode 1426, an identifying unit 1460, and a linking unit 1429. The identifying unit 1460 includes six components 1468a, 1468b, 1468c, 1468d, 1468e, and 1468f. In this embodiment, one side of the linking unit 1429 connects to one end of each of the components 1468a, 1468b, 1468c, 1468d, 1468e, and 1468f, and the other side of the linking unit 1429 connects to the reference electrode 1424 which further connects to the sensing electrode 1426, and therefore the linking unit 1429 provides a common ground for the six components 1468a, 1468b, 1468c, 1468d, 1468e, and 1468f and the sensing electrode 1426.

Although each of the identifying units shown in FIGS. 8-14 has six components, the present invention is not limited to the number of the components. It should be understood that the number and the location of the grooves in the identifying unit can be defined by the designer according to practical applications to compose a plurality of different identification codes. For example, 2^{N}-1 identification codes can be composed for an identifying unit with N components (i.e. the predetermined region of the insulating substrate is divided into N subregions). In other words, each component (subregion) can have two statuses: with and without the groove, and therefore the statuses and the locations of the components can constitute multiple identification codes. Accordingly, the identifying unit can be configured to characterize the biochemical test strips from different batches or with different functions. In another embodiment, the components of the identifying unit can have channels, holes, or indentations, which can either penetrate or not penetrate the insulating substrate. As long as the measurement device can identify the identification code represented by the identifying unit, the present invention is not limited to the shape and size of the groove(s).

FIG. 15 is a block diagram of a biochemical test system 1500 according to an embodiment of the present invention, including a biochemical test strip 1510 and a measurement device 1530. The biochemical test strip 1510 includes a working electrode 1522, a reference electrode 1524, a sensing electrode 1526, and an identifying unit 1560, wherein the identifying unit 1560 includes at least one component, as described above. For example, in the embodiment shown in FIG. 15, the identifying unit 1560 includes six components, and one of the six components includes a groove 1562. The measurement device 1530 includes a connector 1540 and a microprocessor 1550 coupled to the connector 1540. A digital data 1555, for example, testing parameters, detection modes or other information, are built in the microprocessor 1550. The working electrode 1522, the reference electrode 1524, the sensing electrode 1526, and the identifying unit 1560 electrically connect to the measurement 1530 through the connector 1540.

When the biochemical test strip 1510 is connected to the connector 1540, a loop is formed between the sensing electrode 1526 and the connector 1540 to initiate the microprocessor 1550 of the measurement device 1530. Furthermore, since the components of the identifying unit 1560 have different statuses respectively (for example, with or without the groove), the connection between the connector 1540 and the identifying unit 1560 has different configurations, whereby a signal of an identification code represented by the identifying unit 1560 can be generated and transmitted to the microprocessor 1550. The microprocessor 1550 can identify the signal and then choose testing parameters or a test mode from the digital data 1555 corresponding to the signal for execution. The measurement device 1530 further includes a monitor 1570 to display each measurement result, and a power source 1565 to provide necessary power. In another embodiment, the monitor 1570 and the power source 1565 can be external devices, not included in the measurement device 1530.

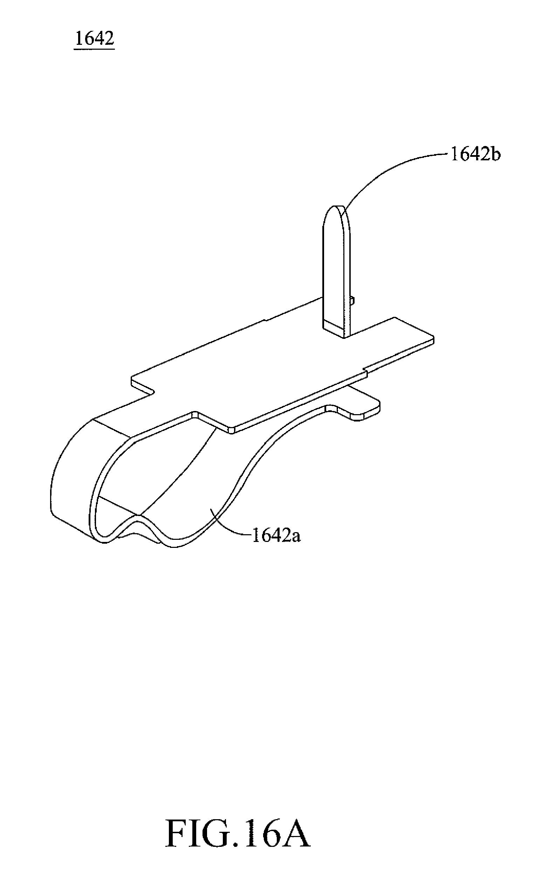

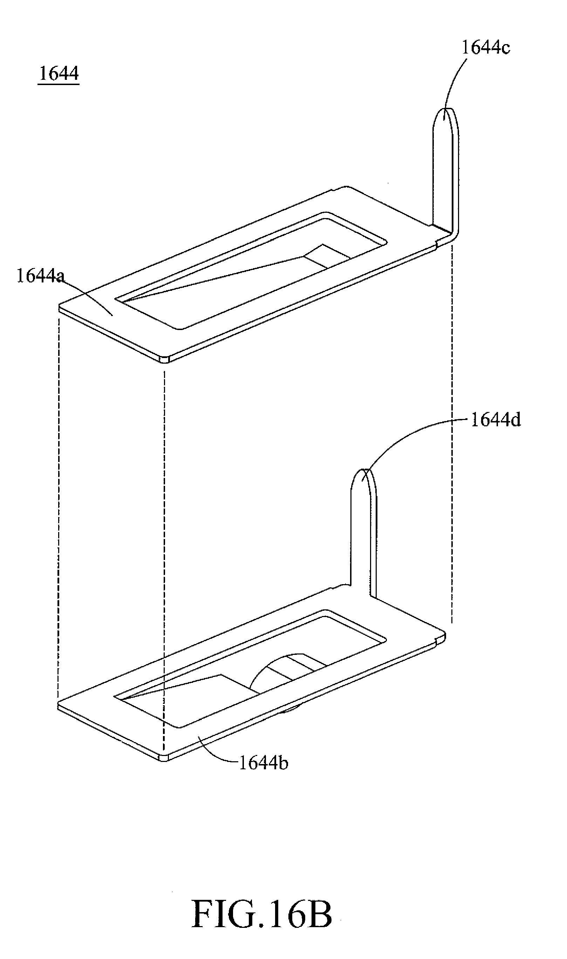

In an embodiment, the connector 1540 includes a plurality of detecting units and at least one sensing unit. As shown in FIG. 16A, the detecting unit 1642 of the connector 1540 is configured to detect the electrical variation between the electrodes on the biochemical test strip and transmit the detected data to the related circuit system. For example, the detecting unit 1642 includes a contact part 1642a and a conducting terminal 1642b. The contact part 1642a electrically contacts with the biochemical test strip to detect the electrical variation between the electrodes, and the detected variation is transmitted to the circuit system through the conducting terminal 1642b to proceed the subsequent signal processing and obtain the measurement result. The number of the sensing unit 1644 corresponds to the number of the components of the identifying unit, i.e. the number of the subregions divided in the predetermined region of the insulating substrate. In other words, the arrangement of the sensing unit 1644 in the connector 1540 corresponds to the location of the identifying unit in the biochemical test strip, for electrically coupling to each component of the identifying unit. The sensing unit 1644 can sense the identifying unit by providing either an open-circuit signal or a short-circuit signal. As shown in FIG. 16B, in one embodiment, the sensing unit 1644 includes two elastic parts 1644a, 1644b and two conducting terminals 1644c, 1644d, wherein the two elastic parts 1644a, 1644b are designed to not touch with each other. However, when the biochemical test strip is inserted into the measurement device 1530, the two elastic parts 1644a, 1644b are compressed (such as by the components without groove) to touch with each other, and therefore a short circuit is formed and then a corresponding signal is generated and outputted to the microprocessor 1550 through the conducting terminals 1644c, 1644d. Namely, based on whether the identifying unit has groove or not and the location of the groove, the sensing unit 1644 of the connector 1540 generates a specific short-circuit signal, and the specific signal is then transmitted to the microprocessor 1550 for the subsequent processing. In other words, as long as the predetermined region of the insulating substrate, which is used to form the identifying unit, has identifiable and patterned structure such that the sensing unit 1644 can generate a corresponding signal based on the statuses of the components (such as with or without the grooves), the microprocessor can select the corresponding mode for the subsequent signal processing based on the corresponding signal.

In addition, in another embodiment, the elastic parts 1644a, 1644b of the sensing unit 1644 are designed to touch each other. Therefore, when the biochemical test strip is inserted into a measurement device, the two elastic parts 1644a, 1644b are pushed away from each other to form an open circuit, and a corresponding signal is generated and outputted to the microprocessor 1550 through the conducting terminals 1644c, 1644d for proceeding the subsequent processing. In other words, as long as the predetermined region of the insulating substrate has an identifiable and patterned structure such that the sensing unit 1644 can generate a corresponding signal based on the status of the individual component (such as with or without the groove), the microprocessor is capable of selecting the corresponding mode for processing based on the received signal. It should be noted that the shapes of both the detecting unit and the sensing unit are not limited to the above embodiments, but may cover other shapes which can achieve the above-described functions. It should be noted that every subregions divided in the predetermined region of the insulating substrate preferably have the same size, or alternatively, can have different sizes due to the different structures of the sensing unit of the connector or the different design needs. Namely, as long as each components of the identifying unit can match with the sensing unit, the present invention is not limited to the size, shape, and depth of desired groove to be formed.

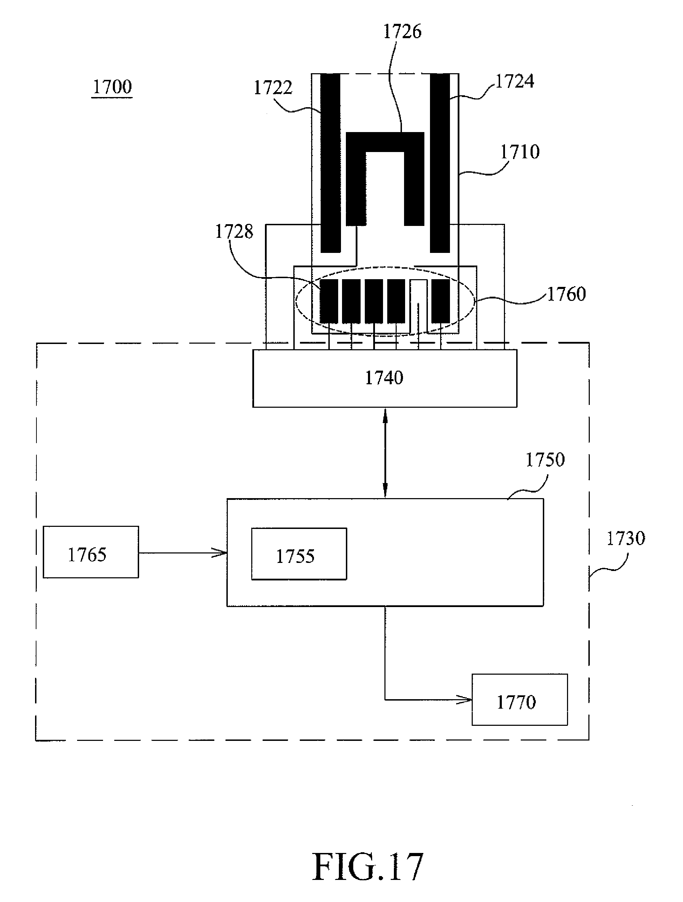

FIG. 17 is a block diagram of a biochemical test system 1700 according to an embodiment of the present invention, including a biochemical test strip 1710 and a measurement device 1730. The biochemical test strip 1710 includes a working electrode 1722, a reference electrode 1724, a sensing electrode 1726, and an identifying unit 1760 with a pattern code 1728, wherein the identifying unit 1760 includes at least one component, as described above. The measurement device 1730 includes a connector 1740, a microprocessor 1750 with digital data 1755, a display 1770, and a power 1765, wherein the functions of these elements of the measurement device 1730 are same as the measurement device 1530 shown in FIG. 15, so the detail description thereof is omitted. Comparing with the test strip shown in FIG. 15, the identifying unit 1760 of the biochemical test strip 1710 includes the pattern code 1728 comprised of conductive material, and therefore can electrically connect to the measurement device 1730 directly through the connector 1740. Therefore, an open-circuit signal corresponding to the groove part of the identifying unit 1760 can be identified by the microprocessor 1750 for proceeding the subsequent signal processing.

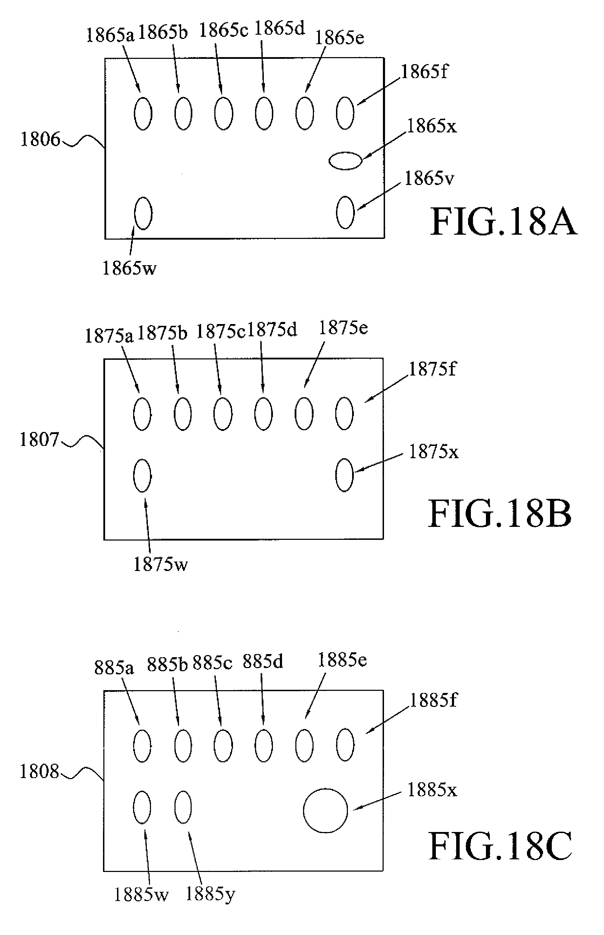

The design of the connectors 1540 and 1740 in FIGS. 15 and 17 can be varied according to different application needs. FIGS. 18A, 18B, and 18C are illustrative diagrams of the connectors 1806, 1807, and 1808 of the biochemical test strips 1100, 1300, and 1400 shown in FIGS. 11,13 and 14 respectively. In one embodiment, the connector of the present invention includes at least the connecting terminals corresponding to the identifying unit, and, through the electrical coupling between the connecting terminals and the identifying unit, the connector can receive a signal corresponding to the identification code represented by the identifying unit.

Referring to FIG. 18A, the connector 1806 includes six identifying terminals 1865a, 1865b, 1865c, 1865d, 1865e, and 1865f, which correspond to the six components 1168a, 1168b, 1168c, 1168d, 1168e, 1168f of the biochemical test strip 1100 respectively. The connector 1806 further includes a ground terminal 1865x corresponding to the linking unit 1129, a measuring terminal 1865w corresponding to the working electrode 1122, and a reference terminal 1865v corresponding to the reference electrode 1124. Referring to FIG. 18B, the connector 1807 includes six identifying terminals 1875a, 1875b, 1875c, 1875d, 1875e, and 1875f, which correspond to the six components 1368a, 1368b, 1368c, 1368d, 1368e, 1368f of the biochemical test strip 1300 respectively. The connector 1807 further includes a ground terminal 1875x corresponding to the reference electrode 1324, and a measuring terminal 1875w corresponding to the working electrode 1322. Referring to FIG. 18C, the connector 1808 includes six identifying terminals 1885a, 1885b, 1885c, 1885d, 1885e, and 1885f, which correspond to the six components 1468a, 1468b, 1468c, 1468d, 1468e, 1468f of the biochemical test strip 1400 respectively. The connector 1808 further includes a ground terminal 1885x corresponding to both the reference electrode 1424 and one end of the sensing electrode 1426, a measuring terminal 1885w corresponding to the working electrode 1422, and a sensing terminal 1885y corresponding to the other end of the sensing electrode 1426.

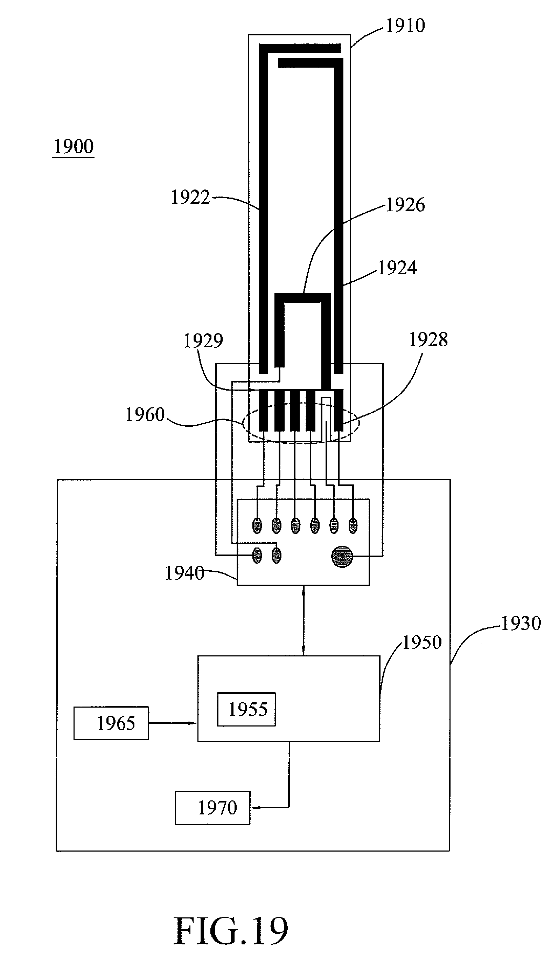

FIG. 19 is a block diagram of a biochemical test system 1900 according to an embodiment of the present invention, including a biochemical test strip 1910 and a measurement device 1930. The biochemical test strip 1910 includes a working electrode 1922, a reference electrode 1924, a sensing electrode 1926, and an identifying unit 1960 with a pattern code 1928, and a linking unit 1929. In this embodiment, the identifying unit 1960 includes six components, and one of the six components includes a groove. One side of the linking unit 1929 connects to one end of each component, and the other side of the linking unit 1929 connects to the sensing electrode 1926, for providing a common ground for the components and the sensing electrode 1926. The measurement device 1930 includes a connector 1940, a microprocessor 1950 with a digital data 1955, a display 1970, and a power 1965, wherein the functions of these elements of the measurement device 1930 are same as the measurement device 1530 shown in FIG. 15, so the detail description thereof is omitted. The working electrode 1922, the reference electrode 1924, the sensing electrode 1926, and the identifying unit 1960 electrically connect to the measurement 1930 through the connector 1940, so as to generate a signal of an identification code represented by the identifying unit 1960 and then transmit this signal to the microprocessor 1950.

The identifying unit of the present invention is provided for identification and to designate the data built in the measurement device. That is, one of the plurality of testing parameters, detection modes, or other information corresponding to the identifying unit of the biochemical test strip can be selected for the measurement device to perform the test procedure without additional code card. The identifying unit of the present invention can be formed by structurally patterning the predetermined region of the insulating substrate, and then optionally coating the conductive material on the predetermined region. The biochemical test system disclosed in the present invention not only achieves the goal to avoid the use of code card, but also reduces the production cost.

The above illustration is for preferred embodiments of the present invention, is not limited to the claims of the present invention. Equivalent amendments and modifications without departing from the spirit of the invention should be included in the scope of the following claims.

## Claims

1. A biochemical test system, comprising:
a biochemical test strip, comprising an insulating substrate, an electrode system disposed on the insulating substrate, and an identifying unit disposed on one side of the insulating substrate, wherein the identifying unit comprises N components, and at least one of the N components comprising a groove, wherein N is an integer greater than two; and
a measurement device, comprising a microprocessor and a connector, wherein the connector is coupled to the identifying unit for receiving a signal of an identification code corresponding to the identifying unit, and the microprocessor is coupled to the connector for receiving the signal from the connector.

2. The biochemical test system according to claim 1, wherein the identifying unit is formed by a predetermined region of the insulating substrate, the identification code corresponding to the identifying unit is decided according to a location of at least one of the N components comprising the groove in the predetermined region.

3. The biochemical test system according to claim 2, wherein the groove comprises a channel, an indentation, and a hole penetrating the insulating substrate, and comprises a channel, an indentation, and a hole without penetrating the insulating substrate.

4. The biochemical test system according to claim 2, wherein the predetermined region of the insulating substrate is divided into N subregions, the N subregions respectively corresponding to the N components, wherein each of the subregions is a desired location of the groove; and
wherein the identifying unit further comprises a pattern code, and the pattern code and the N subregions together form the N components, the pattern code comprising a conductive material.

5. The biochemical test system according to claim 1, wherein the connector comprises at least a sensing unit and the number of the sensing unit corresponds to the N components.

6. The biochemical test system according to claim 5, wherein the sensing unit senses the identifying unit by providing a short circuit or an open circuit whereby the signal is generated.

7. The biochemical test system according to claim 1, further comprising a linking unit, one side of the linking unit connecting with one end of each of the N components.

8. The biochemical test system according to claim 7, wherein the electrode system comprises a working electrode, a reference electrode, and a sensing electrode, and the sensing electrode connects with one side of the linking unit.

9. The biochemical test system according to claim 1, wherein a plurality set of correction parameters are built in the microprocessor, and the microprocessor selects one set of the correction parameters to calibrate the biochemical test system according to the received signal.

10. The biochemical test system according to claim 1, wherein the electrode system comprises a working electrode, a reference electrode, and a sensing electrode insulated from one another, wherein the sensing electrode is provided for detecting an electrical connection between the biochemical test strip and the measurement device.

11. A measurement device, for use with a biochemical test strip, the biochemical test strip comprising an insulating substrate, an electrode system disposed on the insulating substrate, and an identifying unit disposed on one side of the insulating substrate, the identifying unit comprising N components, and at least one of the N components comprising a groove, wherein N is an integer greater than two, the measurement device comprising:
a connector, electrically coupled to the identifying unit, for receiving a signal of an identification code corresponding to the identifying unit; and
a microprocessor, coupled to the connector, for receiving the signal from the connector.

12. The measurement device according to claim 11, wherein the identifying unit is formed by a predetermined region of the insulating substrate, the identification code corresponding to the identifying unit is decided according to a location of at least one of the N components comprising the groove in the predetermined region.

13. The measurement device according to claim 12, wherein the connector comprises at least a sensing unit, and the sensing unit senses the identifying unit by providing a short circuit or an open circuit whereby the signal is generated.

14. The measurement device according to claim 12, wherein the identifying unit further comprises a pattern code, and the pattern code and the N subregions together form the N components, the pattern code comprising a conductive material.

15. The measurement device according to claim 11, wherein the biochemical test strip further comprises a linking unit, one side of the linking unit connecting with one end of each of the N components, and wherein the connector comprises connecting terminals corresponding to the N components, and the connecting terminals electrically couple to the N components.

16. A biochemical test strip, comprising:
an insulating substrate;
an electrode system disposed on the insulating substrate; and
an identifying unit disposed on a predetermined region of the insulating substrate;
wherein the identifying unit comprises N components, N is an integer greater than 2, at least one of the N components comprises a groove, and an identification code for the biochemical test strip is decided according to a location of the groove.

17. The biochemical test strip according to claim 16, wherein the N components generate 2^{N}-1 identification codes.

18. The biochemical test strip according to claim 16, wherein the predetermined region of the insulating substrate is divided into N subregions respectively corresponding to the N components, each of the subregions being a desired location of the groove, wherein the identification code for the biochemical test strip is decided according to the location of the groove in the predetermined region.

19. The biochemical test strip according to claim 18, wherein the groove comprises a channel, an indentation, or a hole penetrating the insulating substrate, and comprises a channel, an indentation, or a hole without penetrating the insulating substrate.

20. The biochemical test strip according to claim 19, wherein the identifying unit further comprises a pattern code, and the pattern code and the N subregions together form the N components, the pattern code comprising a conductive material.

21. The biochemical test strip according to claim 16, further comprising:
an insulating layer, covering a part of the electrode system, wherein a part of the electrode system uncovered by the insulating layer defines a reaction area with an opening;
a cover, disposed on the insulating layer, for covering the reaction area, the cover having a vent corresponding to the reaction area; a reaction layer, disposed in the reaction area, the reaction layer comprising an oxidoreductase;
wherein the electrode system comprises a working electrode, a reference electrode, and a sensing electrode insulated from one another, and the reaction layer at least covers the working electrode.
